# EUROPEAN PATENT APPLICATION

(11) **EP 3 225 231 A1**
(43) Date of publication of application: **04.10.2017**
(21) Application number: 16173267.2
(22) Date of filing: 07.06.2016
(51) Int. Cl.: A61K 8/36, A61K 8/365, A61Q 5/02, A61Q 5/04, A61Q 5/06

(54) **COSMETIC HAIRCARE PRODUCT FOR THE STRAIGHTENING OF HAIR IN SHAMPOO FORMAT**

(30) Priority: 28.03.2016 BR 102016006714
(71) Applicant: Sweet Distribuidora, Importacao e Exportacao de Cosmeticos Ltda. - ME, 03061000 Sao Paulo (BR)
(72) Inventor: Rodrigues Dos Santos, Cassio, 03323-040 São Paulo (BR)
(74) Representative: Elzaburu S.L.P.

(57) **Abstract**

A cosmetic haircare product for the straightening of hair in shampoo format, belonging to the technical field of cosmetics, is disclosed. The shampoo is ready to be used in a single step, and comprises: Water, Sodium Laureth Sulphate, Lactic Acid, Glycolic Acid, Glyoxylic Acid, Cocamide DEA, Cocamidopropyl Betaine, Salicylic Acid, Glycol Distearate, Thioctic acid, Perfume (fragrance), Mica CI 77019, CI 77891, D-Limonene, Linalool, Propylene glycol, Hexyl cinnamal, Sodium hyaluronate, Butylphenyl methylpropional, DMDM hydantoin and Phenoxyethanol. Its method of use is also disclosed.

## Description

### Field of the Invention

The present invention relates to a cosmetic product. More specifically it relates to a product for the straightening of hair, which has been improved for presentation in hair shampoo format.

### State of the art

Generally, the physical format of products for the straightening of hair consists of creams or emulsive lotions consisting of base (alkaline) components such as: ammonium thioglycolate, monoethanolamine thioglycolate, thiolactate, thiomethacrylate, etc., with a pH of between 9.0 and 9.5, requiring neutralisation by means of a second product; and hydroxides such as sodium hydroxide, lithium hydroxide and guanidine carbonate, all these with a pH of between 13 and 14.

It is important to stress that the thio and hydroxide families are incompatible, and once the straightening has been performed with any product from one of these families, it is not possible to apply the other family without causing a chemical reaction in the hair. These products modify permanently the structure of the strands and once applied, the other family can only be applied where the first family has not been deposited, and it is habitual to cut the hair in order to make possible a change in the product applied.

There exists yet another incompatibility with other cosmetic haircare treatments, such as the application of dyes to the hair. Hair which has been straightened with hydroxides cannot be dyed permanently with ethanolamines, as this combination causes a chemical reaction.

Another problem facing the users of this type of straighteners is the health of the strands of hair. The alkaline pH of these formulations removes cuticular layers (keratin) from the strands, rendering them fragile, sensitive, dry and brittle, thus lacking shine, softness and lightness and making necessary the application of other cosmetic haircare products to replace the lost keratin.

There exist other types of straighteners which are not thio- or hydroxide-based and which may be comprised of cysteamine hydrochloride, carbocisteine, mercaptopropionic acid, among others.

The problem currently facing all these products (apart from that stated above) is that similar means of application are used; either with creams or with emulsive or gelled lotions, and furthermore, the time involved in their application, this being 2.5 hours on average, and the different steps entailed in the straightening kit, between 2 and 4 stages (different products for use in the straightening process) is another question to be borne in mind.

### Summary

The object of the present invention patent is to provide a cosmetic haircare product for the straightening of hair, which overcomes the drawbacks and problems of current straighteners.

The solution is based on that the present inventors have found that a composition such as that disclosed in detail below, can be used as a haircare product for the straightening of hair, in shampoo format, which overcomes the problems concerning its application mentioned above and, in addition the processing time of habitual straighteners is considerably reduced. Therefore, this product is easy to use and it can be used in a single step. Also, the cost of every application is lower than that of the existing products.

### Detailed description of the invention

Therefore, in view of the aforementioned problems and with the resolution to overcome the same, and aiming to fulfil the related goals, the following improved cosmetic haircare product for the straightening of hair, in shampoo formatwas developed.

This product comprises:
Water, Sodium Laureth Sulphate, Lactic Acid, Glycolic Acid, Glyoxylic Acid, Cocamide DEA, Cocamidopropyl Betaine, Salicylic Acid, Glycol Distearate, Thioctic acid, Perfume (fragrance), Mica CI 77019, CI 77891, D-Limonene, Linalool, Propylene glycol, Hexyl cinnamal, Sodium hyaluronate, Butylphenyl methylpropional, DMDM hydantoin and Phenoxyethanol.

This composition and the method of use of the cosmetic haircare product for the straightening of hair, in shampoo format, presents advantages in comparison with habitual straighteners.

Specifically, the present straightening shampoo presents a much more rapid method of application (approximately 4 minutes) when compared with the other presentations of straighteners existing on the market, such as creams, emulsive lotions or gels, which may take from 20 to 40 minutes for application of the product to the strands.

As previously indicated, the invention refers to a cosmetic haircare product for the straightening of hair, in shampoo format, which comprises:

| w.t % | INCINAME | CAS |
|---|---|---|
| Balance | Water | 7732-18-5 |
| 8 to 16 | Sodium Laureth Sulfate | 9004-82-4 |
| 5 to 15 | Lactic Acid | 50-21-5 |
| 5 to 15 | Glycolic Acid | 79-14-1 |
| 5 to 15 | Glyoxylic Acid | 298-12-4 |
| 1 to 11 | Cocamide DEA | 68603-42-9 |
| 1 to 11 | Cocamidopropyl Betaine | 61789-40-0 |
| 0.5 to 1.50 | Salicylic Acid | 69-72-7 |
| 0.5 to 1.50 | Glycol Distearate | 627-83-8 |
| 0.5 to 1.50 | Thioctic acid | 1077-28-7 |
| 0.5 to 1.50 | Perfume (fragrance) | Cosing |
| 0.1 to 1 | Mica CI 77019 | 12001-26-2 |
| 0.06 to 1 | CI 77891 | 13463-67-7 |
| 0.04 to 1 | D-Limonene | 5989-27-5 |
| 0.03 to 1 | Linalool | 78-70-6 |
| 0.09 to 1 | Propylene glycol | 57-55-6 |
| 0.02 to 1 | Hexyl cinnamal | 80-54-6 |
| 0.09 to 1 | Sodium hyaluronate | 9067-32-7 |
| 0.01 to 1 | Butylphenyl methylpropional | 118-58-1 |
| 0.09 to 1 | DMDM hydantoin | 6440-58-0 |
| 0.09 to 1 | Phenoxyethanol | 122-99-6 |

These components are combined to yield a straightening product in shampoo format, suited for use in a single step.

In a preferred embodiment, the shampoo comprises:

| w.t % | INCINAME | CAS |
|---|---|---|
| Balance | Water | 7732-18-5 |
| 8 to 16 | Sodium Laureth Sulfate | 9004-82-4 |
| 5 to 15 | Lactic Acid | 50-21-5 |
| 5 to 15 | Glycolic Acid | 79-14-1 |
| 5 to 15 | Glyoxylic Acid | 298-12-4 |
| 1 to 11 | Cocamide DEA | 68603-42-9 |
| 1 to 11 | Cocamidopropyl Betaine | 61789-40-0 |
| 0.5 to 1.50 | Salicylic Acid | 69-72-7 |
| 0.5 to 1.50 | Glycol Distearate | 627-83-8 |
| 0.5 to 1.50 | Thioctic acid | 1077-28-7 |
| 0.5 to 1.50 | Perfume (fragrance) | Cosing |
| 0.1 to 1 | Mica CI 77019 | 12001-26-2 |
| 0.09 to 1 | CI 77891 | 13463-67-7 |
| 0.09 to 1 | D-Limonene | 5989-27-5 |
| 0.09 to 1 | Linalool | 78-70-6 |
| 0.09 to 1 | Propylene glycol | 57-55-6 |
| 0.09 to 1 | Hexyl cinnamal | 80-54-6 |
| 0.09 to 1 | Sodium hyaluronate | 9067-32-7 |
| 0.09 to 1 | Butylphenyl methylpropional | 118-58-1 |
| 0.09 to 1 | DMDM hydantoin | 6440-58-0 |
| 0.09 to 1 | Phenoxyethanol | 122-99-6 |

In a more preferred embodiment, the shampoo comprises 12.65 wt. % of sodium laureth sulphate, 10 wt. % lactic acid, 10 wt. % glycolic acid, 15 wt. % glyoxylic acid, 3 wt.% cocamide DEA, 3 wt.% cocamidopropyl betaine, 1 % salicylic acid, 0.75 wt. % glycol distearate, 1 wt. % thioctic acid, 0.855 wt. % perfume (fragrance), 0.234 wt. % mica CI 77019, 0.066 wt. % CI 77891, 0.043 wt. % D-Limonene, 0.036 wt. % linalool, 1 wt. % propylene glycol, 0.024 wt. % hexyl cinnamal, 1 wt. % sodium hyaluronate, 0.013 wt. % butylphenyl methylpropional, 0.09 wt. % DMDM hydantoin and 0.09 wt. % phenoxyethanol.

The manufacturing process consists in blending and homogenising all the ingredients in an appropriate reactor.

The final product presents the following characteristics:

| | |
|---|---|
| Appearance: | Viscous liquid |
| Colour: | Pearly |
| Aroma: | Characteristic |
| Density: | 1.20 g/mL |
| pH: | Up to 2.00 |

The product is presented in packages of different sizes, between 50g and 1 kg, and also in bulk packs of 50 kg to 1000 kg.

### METHOD OF USE:

1. Moisten the hair.
2. Apply a sufficient quantity of the Straightening Shampoo to cover the entirety of the hair, and massage, without adding water.
3. Allow to act for 20 minutes.
4. Rinse completely.
5. Dry the hair 100%.
6. Iron each lock of hair from 7 to 15 times.

Subsequent to ironing, the hair treatment is complete.

Within the scope of its basic composition, described above, the cosmetic haircare product for the straightening of hair, in shampoo format which is the object of the present patent, may present quantitative and/or qualitative modifications without departing from the scope of protection requested.

## Claims

1. Straightening shampoo which comprises Water, Sodium laureth sulphate, lactic acid, glycolic acid, glyoxylic acid, cocamide DEA, cocamidopropyl betaine, salicylic acid, glycol distearate, thioctic acid, perfume (fragrance), mica CI 77019, CI 77891, D-Limonene, linalool, propylene glycol, hexyl cinnamal, sodium hyaluronate, butylphenyl methylpropional, DMDM hydantoin and phenoxyethanol.

2. Straightening shampoo according to claim 1, which contains 8 to 16 wt. % of sodium laureth sulphate, 5 to 15 wt. % lactic acid, 5 to 15 wt. % glycolic acid, 5 to 15 wt. % glyoxylic acid, 1 to 11 cocamide DEA, 1 to 11 wt.% cocamidopropyl betaine, 0.5 to 1.50 % salicylic acid, 0.5 to 1.5 wt. % glycol distearate, 0.5 to 1.5 wt. % thioctic acid, 0.5 to 1.5 wt. % perfume (fragrance), 0.06 to 1 wt. % mica CI 77019, 0.09 to 1 wt. % CI 77891, 0.04 to 1 wt. % D-Limonene, 0.03 to 1 wt. % linalool, 0.09 to 1 wt. % propylene glycol, 0.02 to 1 wt. % hexyl cinnamal, 0.09 to 1 wt. % sodium hyaluronate, 0.01 to 1 wt. % butylphenyl methylpropional, 0.09 to 1 wt. % DMDM hydantoin and 0.09 to 1 wt. % phenoxyethanol and the rest being water

3. Straightening shampoo according to claim 1, which contains 8 to 16 wt. % of sodium laureth sulphate, 5 to 15 wt. % lactic acid, 5 to 15 wt. % glycolic acid, 5 to 15 wt. % glyoxylic acid, 1 to 11 cocamide DEA, 1 to 11 wt.% cocamidopropyl betaine, 0.5 to 1.50 % salicylic acid, 0.5 to 1.5 wt. % glycol distearate, 0.5 to 1.5 wt. % thioctic acid, 0.5 to 1.5 wt. % perfume (fragrance), 0.1 to 1 wt. % mica CI 77019, 0.09 to 1 wt. % CI 77891, 0.09 to 1 wt. % D-Limonene, 0.09 to 1 wt. % linalool, 0.09 to 1 wt. % propylene glycol, 0.09 to 1 wt. % hexyl cinnamal, 0.09 to 1 wt. % sodium hyaluronate, 0.09 to 1 wt. % butylphenyl methylpropional, 0.09 to 1 wt. % DMDM hydantoin and 0.09 to 1 wt. % phenoxyethanol and the rest being water.

4. Straightening shampoo according to claims 1 or 2, which comprises comprises 12.65 wt. % of sodium laureth sulphate, 10 wt. % lactic acid, 10 wt. % glycolic acid, 10 wt. % glyoxylic acid, 3 wt.% cocamide DEA, 3 wt.% cocamidopropyl betaine, 1 % salicylic acid, 0.75 wt. % glycol distearate, 1 wt. % thioctic acid, 0.855 wt. % perfume (fragrance), 0.234 wt. % mica CI 77019, 0.066 wt. % CI 77891, 0.043 wt. % D-Limonene, 0.036 wt. % linalool, 1 wt. % propylene glycol, 0.024 wt. % hexyl cinnamal, 1 wt. % sodium hyaluronate, 0.013 wt. % butylphenyl methylpropional, 0.09 wt. % DMDM hydantoin and 0.09 wt. % phenoxyethanol and the rest being water.

5. Method for straightening the hair, comprising the steps of:
a) Moistening the hair;
b) Applying the straightening shampoo of any of claims 1 to 4, to cover the entirety of the hair, and massaging, without adding water, allowing it to act for 20 minutes;
c) Rinsing completely;
d) Drying completely the hair;
e) Ironing each lock of hair from 7 to 15 times.
